# EUROPEAN PATENT SPECIFICATION

(11) **EP 1 729 656 B1**
(45) Date of publication and mention of the grant of the patent: **06.11.2013**
(21) Application number: 05723928.7
(22) Date of filing: 25.02.2005
(51) Int. Cl.: A61B 17/32, A61B 17/3201

(54) **ULTRASONIC SURGICAL SHEARS AND TISSUE PAD FOR SAME**
CHIRURGISCHE ULTRASCHALLSCHERE UND ZUGEHÖRIGE GEWEBEUNTERLAGE
CISEAUX CHIRURGICAUX A ULTRASONS ET LEUR TAMPON DE TISSU

(30) Priority: 27.02.2004 US 548301 P; 24.02.2005 US 65378
(43) Date of publication of application: 13.12.2006
(60) Divisional application: 12163422.4
(73) Proprietor: ETHICON ENDO-SURGERY, INC., Cincinnati, OH 45242-2839 (US)
(72) Inventor: HOUSER, Kevin L., Springboro, Ohio 45066 (US); NOSCHANG, Sarah A., Mason, Ohio 45040 (US); NEUENFELDT, Steven, Pleasanton, California 94566 (US); FALLER, Craig N., Milford, Ohio 45150 (US); VAITEKUNAS, Jeffrey J., Lakeville, Minnesota 55044-6611 (US)
(74) Representative: Tunstall, Christopher Stephen
(86) International application number: PCT/US2005/006272
(87) International publication number: WO 2005/084250

(56) References cited:
- WO-A-2004/012615
- GB-A- 2 348 810
- US-A- 3 101 715
- US-A- 3 503 397
- US-A- 6 129 735
- US-A- 6 139 561
- US-A1- 2002 128 645
- US-B1- 6 312 430
- US-B2- 6 468 286
- DUPONT KEVLAR: '149 Fiber, dim. 12mum Material Data Sheet' MATWEB, [Online] 1996, XP003015289 Retrieved from the Internet: <URL:http://www.matweb.com/search/SpecificM aterial/Print.asp?bassnum=PCF003>
- 'Properties of Polyimide {thermoset, unfilled}. Datasheet' EFUNDA POLYMERS, [Online] XP003015290 Retrieved from the Internet: <URL:http://www.efunda.com/materials/polyme rs/properties/polymer_datasheet.cfm?MajorID =PI&MinorID=1>
- 'Fluoropolymer Comparison - Typical Properties, Comparison Chart' DUPONT, [Online] XP003015291 Retrieved from the Internet: <URL:http://www2.dupont.com/Teflon_Industri al_US/tech_info/techinfo_compare.html>

## Description

**Field of the Invention**

The present invention is related generally to surgical instruments, and more particularly to an ultrasonic surgical shears and to a tissue pad for an ultrasonic surgical shears.

**Background of the Invention**

Ultrasonic surgical instruments are known which include an ultrasonic surgical shears having an ultrasonic surgical blade, a clamping arm operable to open and close toward the blade, and a polytetrafluoroethylene tissue pad which is attached to the clamping arm and which includes a clamping surface. The clamping arm exerts a clamping force on a blood vessel which is positioned between the clamping surface of the tissue pad and the blade. The result of the ultrasonically-vibrating ultrasonic surgical blade and the clamping force on the blood vessel is a coaptation of the blood vessel (a bringing together of the walls of the blood vessel), a transection (a cutting) of the coapted blood vessel, and a coagulation (a sealing) of the coapted cut ends of the blood vessel. At the completion of a tissue transection, the ultrasonically-vibrating ultrasonic surgical blade contacts and cuts away some of the polytetrafluoroethylene tissue pad because of the frictional abrasion and frictional heat generated by the blade vibrating against the tissue pad. Exemplary devices are described in U.S. Patent Serial Numbers 5,322,055 and 6,325,811. US-A-6129735 discloses an ultrasonic treatment appliance comprising a distal probe part formed as an integral part of or independently of the probe at the distal end of the probe, a jaw opposed to the distal probe part so that the jaw can open or close freely, and a thin chip located on the jaw or, the clamping side of the clamping portion of the jaw on which the clamping portion touches a living tissue, or an elastic member interposed between the supporting portion and clamping portion of the jaw.

Still, scientists and engineers continue to seek improved ultrasonic surgical shears and improved tissue pads for ultrasonic surgical shears.

**Summary of the Invention**

A first embodiment of an ultrasonic-surgical-shears tissue pad of the invention includes an ultrasonic-surgical-shears tissue pad body as claimed in claim 1, having a base material and at least one filler material which is a different material from the base material. Preferred embodiments are disclosed in the dependent claims.

Several benefits and advantages are obtained from one or more of the embodiments of the invention. Having a tissue pad with a base material and at-least-one filler material allows the base material and the at-least-one filler material to be chosen with a different hardness, stiffness, lubricity, dynamic coefficient of friction, heat transfer coefficient, abradability, heat deflection temperature, and/or melt temperature to improve the wearability of the tissue pad which is important when high clamping forces are employed because tissue pads wear faster at higher clamping forces than at lower clamping forces. Applicants found, in one experiment, that a 15% graphite-filled polytetrafluoroethylene tissue pad showed substantially the same wear with a 3kg (7 pound) clamping force as a 100% polytetrafluoroethylene tissue pad showed with a 0.7kg (1.5 pound) clamping force. Having a flexible clamping arm and/or a flexible tissue pad should also improve the wearability of the tissue pad due to the ability of the flexible member to more evenly distribute the load across the entire surface of the tissue pad.

The present invention has, without limitation, application in straight or curved ultrasonic surgical blades as disclosed in the patents incorporated by reference and further in hand-activated instruments as well as in robotic-assisted instruments.

**Brief Description of the Figures**

FIGURE 1 is a cross-sectional view of a portion of a first embodiment of an ultrasonic-surgical-shears tissue pad of the invention;

FIGURE 2 is a cross-sectional view of a portion of a second embodiment of an ultrasonic-surgical-shears tissue pad of the invention;

FIGURE 3 is a side-elevational view of a first alternate embodiment of the tissue pad of Figure 2;

FIGURE 4 is a side-elevational view of a second alternate embodiment of the tissue pad of Figure 2;

FIGURE 5 is a side-elevational view of a third additional alternate embodiment of the tissue pad of Figure 2;

FIGURE 6 is a schematic side elevational view of a portion of an embodiment of an ultrasonic surgical shears of the invention;

FIGURE 7 is a schematic side elevational view of a portion of an alternate embodiment of an ultrasonic surgical shears of the invention.

**Detailed Description of the Invention**

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is understood that any one or more of the following-described embodiments, examples, etc. can be combined with any one or more of the other following-described embodiments, examples, etc.

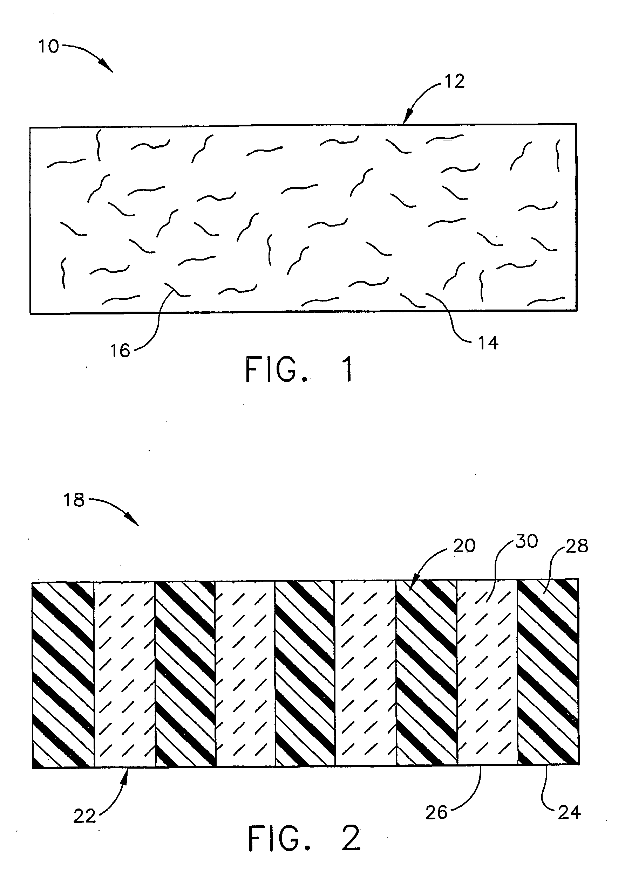

Referring now to the Figures, in which like numerals indicate like elements, Figure 1 illustrates a first embodiment of an ultrasonic-surgical-shears tissue pad 10 of the invention. The ultrasonic-surgical-shears tissue pad 10 has an ultrasonic-surgical-shears tissue pad body 12 including a base material 14 and at least one filler material 16 which is a different material from the base material 14.

In one example of the embodiment of the ultrasonic-surgical-shears tissue pad 10 of Figure 1, the at-least-one filler material 16 has at least one property which has a different value from that of the at-least-one property of the base material 14, wherein the at-least-one property is chosen from the group consisting of: hardness, stiffness, lubricity, dynamic coefficient of friction, heat transfer coefficient, abradability, heat deflection temperature, and melt temperature. In one variation, at least two or more or all of the properties have different values for the base material 14 and the at-least-one filler material 16.

In one illustration of the ultrasonic-surgical-shears tissue pad 10 of Figure 1, the base material 14 has a heat deflection temperature greater than 500 K (500 degrees Fahrenheit). In the same or a different illustration, the base material 14 has a melt temperature greater than 600K (700 degrees Fahrenheit). In the same or a different illustration, the base material 14 has a dynamic coefficient of friction less than 0.3 at pressure-velocity values greater than 4000 kg per meter-second (30,000 pounds per foot-second). In one choice of materials of the ultrasonic-surgical-shears tissue pad 10 of Figure 1, the base material 14 consists essentially of a thermoset plastic material. In one variation, the base material 14 consists essentially of a polyimide material.

In one enablement of the invention, the at-least-one filler material 16 has a hardness which is different than that of the base material 14. In the same or a different enablement, the at-least-one filler material 16 has a stiffness which is different than that of the base material 14. In the same or a different enablement, the at-least-one filler material 16 has a lubricity which is different than that of the base material 14. In the same or a different enablement, the at-least-one filler material 16 has a dynamic coefficient of friction which is different than that of the base material 14. In the same or a different enablement, the at-least-one filler material 16 has a heat transfer coefficient which is different than that of the base material 14. In the same or a different enablement, the at-least-one filler material 16 has an abradability which is different than that of the base material 14. In the same or a different enablement, the at-least-one filler material 16 has a heat deflection temperature which is different than that of the base material 14. In the same or a different enablement, the at-least-one filler material 16 has a melt temperature which is different than that of the base material 14.

In one example of the invention, the at-least-one filler material 16 is chosen from the group consisting of glass, carbon fiber, graphite, metal particles, molybdenum disulfide, a liquid lubricant, a solid material that changes to a more lubricous powder at an increased temperature, a solid that changes to a liquid at an increased temperature, carbon nanotubes, polyphenelene sulfone, polyphenelene sulfide, sumifine powder, boron nitride, polytetrafluoroethylene powder, silicone oil, and an aerogel.

In the same or another example of the invention, the base material 14 is chosen from the group consisting of a plastic, a porous ceramic, a polished ceramic, a self-constructing nanocomposite (a material that is a combination of two or more materials that, when cured, structures itself into a predetermined matrix), a highly crosslinked polytetrafluoroethylene, a metal having a hardness at least as low as tantalum, a fluorinated polyimide, a clay-filled nanocomposite-forming polymer (these are materials that are filled with small amounts of clay material where the clay material combines with the polymer molecule to yield a material with superior properties to the original polymer material such as a clay-filled nylon that exhibits a heat deflection temperature of at least 300 K (100 degrees Fahrenheit) higher than that of the regular nylon material), and a polyimide material. In one variation, the plastic is chosen from the group consisting of a polytetrafluoroethylene and a polyimide. In one modification, substantially 85% of the ultrasonic-surgical-blade tissue pad body 12 consists essentially of the base material 14 and substantially 15% of the ultrasonic-surgical-blade tissue pad body 12 consists essentially of the at-least-one filler material 16, wherein the base material 14 consists essentially of polytetrafluoroethylene, and wherein the at-least-one filler material 16 consists essentially of graphite.

In one expression of the invention, the ultrasonic-surgical-shears tissue pad body 12 includes a base material 14 and at least one filler material 16, wherein the base material 14 is chosen from the group consisting of a plastic, a porous ceramic, a polished ceramic, a self-constructing nanocomposite, a highly crosslinked polytetrafluoroethylene, a metal having a hardness at least as low as tantalum, a fluorinated polyimide, a clay-filled nanocomposite-forming polymer, and a polyimide material.

In one configuration of the invention, not shown, the ultrasonic-surgical-shears tissue pad body consists essentially of a material chosen from the group consisting of a porous ceramic, a polished ceramic, a self-constructing nanocomposite, a highly crosslinked polytetrafluoroethylene, a metal having a hardness at least as low as tantalum, a fluorinated polyimide, a clay-filled nanocomposite-forming polymer, and a polyimide.

In one deployment of the invention, the ultrasonic-surgical-shears tissue pad body 12 includes a base material 14 and at least one filler material 16, wherein the base material 14 consists essentially of a porous polymer, and wherein the at-least-one filler material 16 is chosen from the group consisting essentially of a solid lubricant, a liquid lubricant, and a solid lubricant which changes to a liquid lubricant at an increased temperature.

In one arrangement of the invention, not shown, the ultrasonic-surgical-shears tissue pad body consists essentially of a porous wicking material which upon contact wicks patient body fluids into the ultrasonic-surgical-shears tissue pad body or absorbs water when immersed in a water containing solution such as saline. These materials improve the temperature performance of the tissue pad body by absorbing some of the heat energy to evaporate the water entrapped in the tissue pad body.

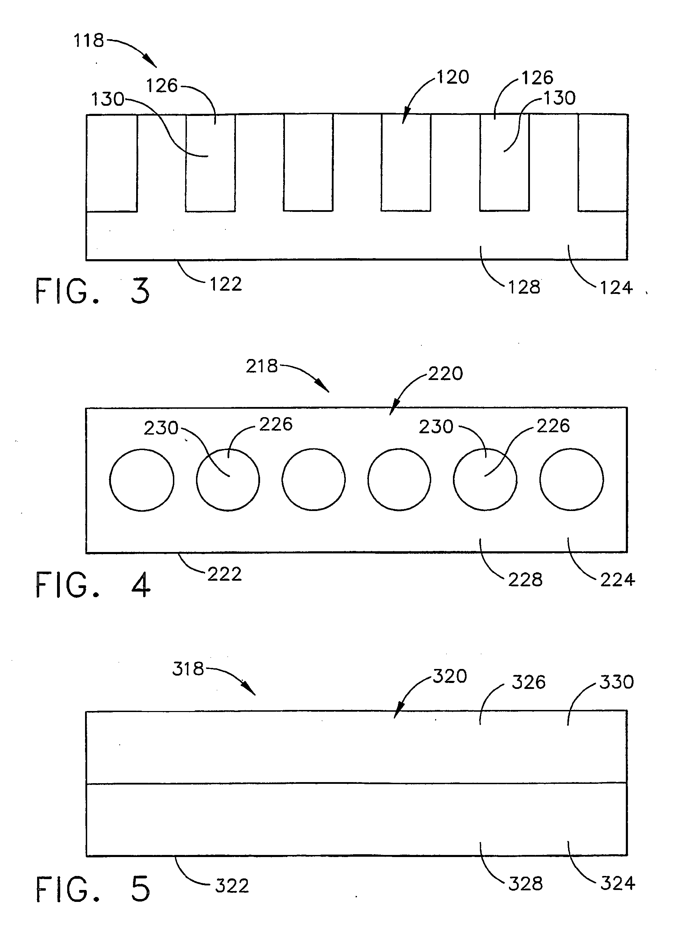

Figure 2 illustrates a second embodiment of an ultrasonic-surgical-shears tissue pad 18 of the invention. The ultrasonic-surgical-shears tissue pad 18 has an ultrasonic-surgical-shears tissue pad body 20 having adjoining first and second regions 24 and 26, wherein the first region 24 includes a first material 28 and wherein the second region 26 includes a second material 30 which is a different material from the first material 28. The above description of the tissue pad 18 of Figure 2 is equally applicable to the tissue pads of Figures 3-5, as can be appreciated by the artisan from the below discussion of the tissue pads of Figures 3-5. In one variation of the tissue pad 18 of Figure 2, the first region 24 consists essentially of the first material 28 and the second region 26 consists essentially of the second material 30. In another variation, the first region 24 includes a base material and at least one filler material, wherein the base material is the first material 28. In the same or a different variation, the second region 26 includes a base material and at least one filler material, wherein the base material is the second material 30.

In one construction of the tissue pad 18 of Figure 2, the interface between the first and second regions 24 and 26 of the tissue pad body 20 is substantially perpendicular to the clamping surface 22 of the tissue pad body 20 as shown in the figure. In another construction, not shown, the interface between the first and second regions is substantially parallel to the clamping surface (this can be visualized by rotating the tissue pad 18 in Figure 2 by ninety degrees. In an additional construction, not shown, the interface is slanted with respect to the clamping surface at an angle between substantially 1 and 89 degrees, as can be appreciated by the artisan.

It is noted that the examples, illustrations, choices of materials, etc. described for the embodiment of the ultrasonic-surgical-shears tissue pad 10 of Figure 1 are equally applicable to the embodiment of the ultrasonic-surgical-shears tissue pad 18 of Figure 2 with the phrase "first material 28" replacing the phrase "base material 14" and with the phrase "second material 30" replacing the phrase "at-least-one filler material 16".

Figure 3 is an exterior side-elevational view of a tissue pad 118 which is a first alternate embodiment to the tissue pad 18 of Figure 2. Tissue pad 118 includes tissue pad body 120 having adjoining first and second regions 124 and 126 as shown in the figure. First region 124 includes a first material 128, and second region 126 includes a second material 130 which is a different material from the first material. In one variation, the clamping surface 122 of the tissue pad body 120 consists essentially of the first material 128 which extends away from the clamping surface 122 toward the second regions 126. In one enablement, the material transversely between the second regions 126 is the first material 128 of the first region 124. In another enablement, not shown, a third region with a third material is disposed transversely between the second regions.

Figure 4 is an exterior side-elevational view of a tissue pad 218 which is a second alternate embodiment to the tissue pad 18 of Figure 2. Tissue pad 218 includes tissue pad body 220 having adjoining first and second regions 224 and 226 as shown in the figure. First region 224 includes a first material 228, and second region 226 includes a second material 230 which is a different material from the first material. In one variation, the clamping surface 222 of the tissue pad body 220 consists essentially of the first material 228 which extends away from the clamping surface 222 toward the second regions 226. In one enablement, the material transversely between the second regions 226 is the first material 228 of the first region 224. In another enablement, not shown, a third region with a third material is disposed transversely between the second regions.

Figure 5 is an exterior side-elevational view of a tissue pad 318 which is a third alternate embodiment to the tissue pad 18 of Figure 2. Tissue pad 318 includes tissue pad body 320 having adjoining first and second regions 324 and 326 as shown in the figure. First region 324 includes a first material 328, and second region 326 includes a second material 330 which is a different material from the first material. In one variation, the clamping surface 322 of the tissue pad body 320 consists essentially of the first material 328 which extends away from the clamping surface 322 toward the second regions 326. In one application, tissue pad 318 improves pad life by the first region 324 being sacrificial and being abraded or melted relatively quickly but having certain properties, such as lubricity, that are desirable. The ultrasonic surgical blade, not shown in Figure 5, moves through the first material 318 and then comes into contact with the second material 330. The second material 330 is selected for properties that make it abrade or melt less than the first material 318.

It is noted that the examples, illustrations, choices of materials, etc. described for the embodiment of the tissue pad 18 of Figure 2 are equally applicable to the embodiments of the tissue pad 118, 218 and 318 of Figures 3-5. Other alternate embodiments to the tissue pad 18 are left to the artisan.

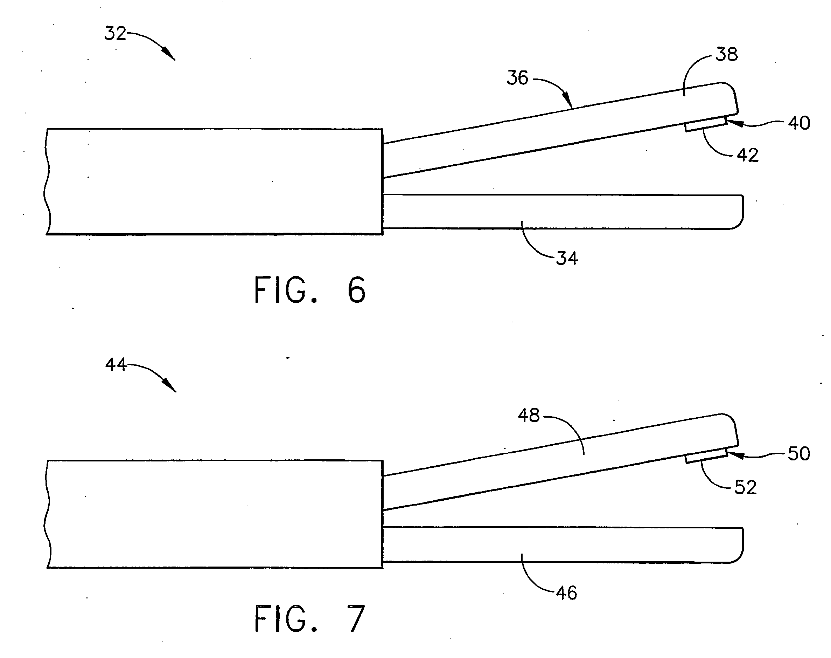

Figure 6 illustrates a first embodiment of an ultrasonic surgical shears 32 of the invention. The ultrasonic-surgical-shears 32 includes an ultrasonic surgical blade 34 and a clamping arm 36 operable to open and close toward the blade 34 and having a transversely and resiliently flexible distal tip 38. By "resiliently flexible distal tip" is meant that the distal tip 38 resiliently flexes during clamping of the clamping arm 36 such as when the ultrasonic-surgical-shears 32 is used to transect and seal a blood vessel, disposed between the clamping surface 42 and the ultrasonic surgical blade 34, whose walls have been coapted by a clamping force applied via the clamping arm 36. In one implementation of the first expression, the ultrasonic surgical shears 32 also includes a tissue pad 40 attached to the clamping arm 36 and having a clamping surface 42, wherein the tissue pad 40 is resiliently flexible in a direction substantially perpendicular to the clamping surface 42. In one illustration of the embodiment of the ultrasonic-surgical-shears 32, the tissue pad 40 includes a base material and at least one filler material as previously described for the tissue pad 10 of Figure 1. In another illustration of the ultrasonic-surgical-shears 32, the tissue pad 40 includes a first material and a second material as previously described for the tissue pad 18, 118, 218 or 318 of Figures 2-5.

Figure 7 illustrates a second embodiment of an ultrasonic surgical shears 44 of the invention. The ultrasonic-surgical-shears 44 includes an ultrasonic surgical blade 46, a clamping arm 48 operable to open and close toward the blade 46, and a tissue pad 50. The tissue pad 50 is attached to the clamping arm 48 and has a clamping surface 52. At least a portion of the tissue pad 50 is resiliently flexible in a direction substantially perpendicular to the clamping surface 52. By "resiliently flexible" is meant that the tissue pad 50 resiliently flexes during clamping of the clamping arm 48 such as when the ultrasonic-surgical-shears 44 is used to transect and seal a blood vessel, disposed between the clamping surface 52 and the ultrasonic surgical blade 46, whose walls have been coapted by a clamping force applied via the clamping arm 48. In one illustration of the embodiment of the ultrasonic-surgical-shears 44, the tissue pad 50 includes a base material and at least one filler material as previously described for the tissue pad 10 of Figure 1. In another illustration of the ultrasonic-surgical-shears 44, the tissue pad 40 includes a first material and a second material as previously described for the tissue pad 18, 118, 218 or 318 of Figures 2-5.

Several benefits and advantages are obtained from one or more of the embodiments of the invention. Having a tissue pad with a base material and at-least-one filler material allows the base material and the at-least-one filler material to be chosen with a different hardness, stiffness, lubricity, dynamic coefficient of friction, heat transfer coefficient, abradability, heat deflection temperature, and/or melt temperature to improve the wearability of the tissue pad which is important when high clamping forces are employed because tissue pads wear faster at higher clamping forces than at lower clamping forces. Applicants found, in one experiment, that a 15% graphite-filled polytetrafluoroethylene tissue pad showed substantially the same wear with a 3kg (7 pound) clamping force as a 100% polytetrafluoroethylene tissue pad showed with a 0.7 kg (1.5 pound) clamping force. Having a flexible clamping arm and/or a flexible tissue pad should also improve the wearability of the tissue pad due to the ability of the flexible member to more evenly distribute the load across the entire surface of the tissue pad.

While the present invention has been illustrated by a description of several embodiments, it is not the intention of the applicants to restrict or limit the scope of the appended claims to such detail. Numerous other variations, changes, and substitutions will occur to those skilled in the art without departing from the scope of the invention. For instance, the ultrasonic surgical shears and the tissue pad of the invention have application in robotic assisted surgery taking into account the obvious modifications of such systems, components and methods to be compatible with such a robotic system. It will be understood that the foregoing description is provided by way of example, and that other modifications may occur to those skilled in the art without departing from the scope of the appended Claims.

## Claims

1. An ultrasonic-surgical-shears tissue pad (10) comprising: an ultrasonic-surgical-shears tissue pad body (12) including a base material (14) and **characterized in that** the tissue pad body includes at least one filler material (16) which is a different material from the base material.

2. The ultrasonic-surgical-shears tissue pad of claim 1, wherein the at-least-one filler material (16) has at least one property which has a different value from that of the at-least-one property of the base material (14), and wherein the at-least-one property is chosen from the group consisting of: hardness, stiffness, lubricity, dynamic coefficient of friction, heat transfer coefficient, abradability, heat deflection temperature, and melt temperature.

3. The ultrasonic-surgical-shears tissue pad of claim 1, wherein the base material (14) has a heat deflection temperature greater than 500 K (500 degrees Fahrenheit).

4. The ultrasonic-surgical-shears tissue pad of claim 1, wherein the base material (14) has a melt temperature greater than 600 K (700 degrees Fahrenheit).

5. The ultrasonic-surgical-shears tissue pad of claim 1, wherein the base material (14) has a dynamic coefficient of friction less than 0.3 at pressure-velocity values greater than 4000 kg per meter-second (30,000 pounds per foot-second).

6. The ultrasonic-surgical-shears tissue pad of claim 1, wherein the base material (14) consists essentially of a thermoset plastic material.

7. The ultrasonic-surgical-shears tissue pad of claim 6, wherein the base material (14) consists essentially of a polyimide material.

8. An ultrasonic surgical shears (32) comprising:
a) an ultrasonic surgical blade (34);
b) a clamping arm operable (36) to open and close toward the blade and having a transversely and resiliently flexible distal tip (38); and
c) a tissue pad (50) according to claim 2 wherein at least a portion of the tissue pad is resiliently flexible in a direction substantially perpendicular to the clamping surface.

9. An ultrasonic surgical shears (44) comprising:
a) an ultrasonic surgical blade (46);
b) a clamping arm (48) operable to open and close toward the blade; and
c) a tissue pad (50) according to claim 2 attached to the clamping arm and having a clamping surface, wherein at least a portion of the tissue pad is resiliently flexible in a direction substantially perpendicular to the clamping surface.

## Patentansprüche

1. Chirurgische Ultraschallscheren-Gewebeunterlage (10), umfassend: einen Ultraschallscheren-Gewebeunterlagenkörper (12) mit einem Grundmaterial (14) und **dadurch gekennzeichnet, dass** der Gewebeunterlagenkörper zumindest ein Füllmaterial (16) aufweist, bei dem es sich um ein anderes Material als das Grundmaterial handelt.

2. Chirurgische Ultraschallscheren-Gewebeunterlage nach Anspruch 1, worin das zumindest eine Füllmaterial (16) zumindest eine Eigenschaft aufweist, die einen anderen Wert als die zumindest eine Eigenschaft des Grundmaterials (14) aufweist und worin die zumindest eine Eigenschaft aus der aus Härte, Steifheit, Gleitfähigkeit, dynamischer Reibungskoeffizient, Wärmeübertragungskoeffizient, Abrasionsfähigkeit, Formbeständigkeitstemperatur und Schmelztemperatur bestehenden Gruppe ausgewählt ist.

3. Chirurgische Ultraschallscheren-Gewebeunterlage nach Anspruch 1, worin das Grundmaterial (14) eine Formbeständigkeitstemperatur von mehr als 500 K (500 Grad Fahrenheit) aufweist.

4. Chirurgische Ultraschallscheren-Gewebeunterlage nach Anspruch 1, worin das Grundmaterial (14) eine Schmelztemperatur von mehr als 600 K (700 Grad Fahrenheit) aufweist.

5. Chirurgische Ultraschallscheren-Gewebeunterlage nach Anspruch 1, worin das Grundmaterial (14) einen dynamischen Reibungskoeffizienten unter 0,3 bei Druck-Geschwindigkeits-Werten von mehr als 4000 kg pro Meter-Sekunde (30.000 Pfund pro Fuß und Sekunde) aufweist.

6. Chirurgische Ultraschallscheren-Gewebeunterlage nach Anspruch 1, worin das Grundmaterial (14) im Wesentlichen aus einem Duroplasten besteht.

7. Chirurgische Ultraschallscheren-Gewebeunterlage nach Anspruch 6, worin das Grundmaterial (14) im Wesentlichen aus einem Polyimid-Material besteht.

8. Chirurgische Ultraschallschere (32), umfassend:
a) eine chirurgische Ultraschallklinge (34);
b) einen Klemmarm (36), der zum Öffnen und Schließen zur Klinge hin betätigt werden kann und eine quer und elastisch flexible distale Spitze (38) aufweist; und
c) eine Gewebeunterlage (50) nach Anspruch 2, worin zumindest ein Teil der Gewebeunterlage in einer zur Klemmfläche im Wesentlichen senkrechten Richtung elastisch flexibel ist.

9. Chirurgische Ultraschallschere (44), umfassend:
a) eine chirurgische Ultraschallklinge (46);
b) einen Klemmarm (48), der zum Öffnen und Schließen zur Klinge hin betätigt werden kann; und
c) eine Gewebeunterlage (50) nach Anspruch 2, die am Klemmarm befestigt ist und eine Klemmfläche aufweist, worin zumindest ein Teil der Gewebeunterlage in einer zur Klemmfläche im Wesentlichen senkrechten Richtung elastisch flexibel ist.

## Revendications

1. Tampon de tissu pour ciseaux chirurgicaux à ultrasons (10), comprenant un corps de tampon de tissu de ciseaux chirurgicaux à ultrasons (12) comprenant une matière de base (14), et **caractérisé en ce que** le corps de tampon de tissu comprend au moins une matière de remplissage (16) qui est une matière différente de la matière de base.

2. Tampon de tissu pour ciseaux chirurgicaux à ultrasons selon la revendication 1, dans lequel ladite au moins une matière de remplissage (16) présente au moins une propriété dont la valeur est différente de celle de ladite au moins une propriété de la matière de base (14), et dans lequel ladite au moins une propriété est choisie dans le groupe comprenant la dureté, la rigidité, le pouvoir lubrifiant, le coefficient de frottement dynamique, le coefficient de transfert de chaleur, l'aptitude à l'abrasion, la température de déflexion à la chaleur et la température de fusion.

3. Tampon de tissu pour ciseaux chirurgicaux à ultrasons selon la revendication 1, dans lequel la matière de base (14) présente une température de déflexion à la chaleur qui est supérieure à 500 K (500 degrés Fahrenheit).

4. Tampon de tissu pour ciseaux chirurgicaux à ultrasons selon la revendication 1, dans lequel la matière de base (14) présente une température de fusion qui est supérieure à 600 K (700 degrés Fahrenheit).

5. Tampon de tissu pour ciseaux chirurgicaux à ultrasons selon la revendication 1, dans lequel la matière de base (14) présente un coefficient de frottement dynamique qui est inférieur à 0,3 à des valeurs de pression-vitesse supérieures à 4000 kg par mètre-seconde (30000 livres par pied-seconde).

6. Tampon de tissu pour ciseaux chirurgicaux à ultrasons selon la revendication 1, dans lequel la matière de base (14) est essentiellement constituée d'une matière plastique thermodurcissable.

7. Tampon de tissu pour ciseaux chirurgicaux à ultrasons selon la revendication 6, dans lequel la matière de base (14) est essentiellement constituée d'une matière de polyimide.

8. Ciseaux chirurgicaux à ultrasons (32), comprenant:
a) une lame chirurgicale à ultrasons (34);
b) un bras de serrage (36) actionnable pour s'ouvrir et se fermer en direction de la lame et présentant une extrémité distale transversalement et élastiquement flexible (38); et
c) un tampon de tissu (50) selon la revendication 2, dans lequel au moins une partie du tampon de tissu est élastiquement flexible dans une direction sensiblement perpendiculaire à la surface de serrage.

9. Ciseaux chirurgicaux à ultrasons (44), comprenant:
a) une lame chirurgicale à ultrasons (46);
b) un bras de serrage (48) actionnable pour s'ouvrir et se fermer en direction de la lame; et
c) un tampon de tissu (50) selon la revendication 2 qui est attaché au bras de serrage et qui présente une surface de serrage, dans lequel au moins une partie du tampon de tissu est élastiquement flexible dans une direction sensiblement perpendiculaire à la surface de serrage.
